# EUROPÄISCHE PATENTSCHRIFT

(11) **EP 3 056 222 B1**
(45) Veröffentlichungstag und Bekanntmachung des Hinweises auf die Patenterteilung: **17.01.2018**
(21) Anmeldenummer: 16450001.9
(22) Anmeldetag: 28.01.2016
(51) Int. Cl.: A61L 2/08, A61L 2/10

(54) **VORRICHTUNG ZUM BESTRAHLEN VON GEGENSTÄNDEN MIT ELEKTROMAGNETISCHER STRAHLUNG**
DEVICE FOR IRRADIATING OBJECTS WITH ELECTROMAGNETIC RADIATION
DISPOSITIF POUR IRRADIER DES OBJETS AVEC RAYONNEMENT ÉLECTROMAGNÉTIQUE

(30) Priorität: 16.02.2015 AT 782015
(43) Veröffentlichungstag der Anmeldung: 17.08.2016
(73) Patentinhaber: SICO Technology GmbH, 9531 Bleiberg-Kreuth (AT)
(72) Erfinder: SIMCIC, Christian, 9582 Latschach (AT); NADRAG, Enrico, 9530 Bad Bleiberg Nr. 59 (AT)
(74) Vertreter: Beer & Partner Patentanwälte KG

(56) Entgegenhaltungen:
- EP-A1- 2 567 713
- EP-A2- 0 818 206
- US-A1- 2011 127 448
- US-A1- 2013 270 445

## Beschreibung

Die Erfindung betrifft eine Vorrichtung zum Bestrahlen von Gegenständen mit elektromagnetischer Strahlung, wie beispielsweise elektromagnetischer Strahlung im Bereich des sichtbaren Lichtes, im IR-Bereich oder im UV-Bereich. Beispielsweise werden Gegenstände, die mit Titandioxid beschichtet sind, mit UV-Strahlung bestrahlt, um der Beschichtung aus Titandioxid, das insbesondere in Form von Partikeln im Nanogrößenbereich (= Nanopulver) vorliegen kann, Eigenschaften zu verleihen, die ein unerwünschtes Anlagern von Feststoffen ("Fouling") an der Oberfläche von Gegenständen, insbesondere das Anwachsen ("Bewuchs") von sessilen Organismen, verhindern. Durch die UV-Bestrahlung werden den mit Titandioxid beschichteten Gegenständen "Antifouling"-Eigenschaften verliehen.

Ein Anwendungsbeispiel ist das Bestrahlen von Fasern, aus denen Filtermatten für Wasserentsalzungsanlagen hergestellt sind. Dabei soll erreicht werden, dass während des Stillstandes von Wasserentsalzungsanlagen kein Bewuchs ("Biofouling", "Biofilmbildung") auf den Filtermatten, die beispielsweise aus mit Titanoxid beschichteten keramischen Fasern bestehen, entsteht.

US 2011/127448 A1 offenbart eine Vorrichtung zum Bestrahlen von flüssigen Mischmaterialien, wie Blut, umfassend wenigstens eine UV-Quelle, die UV-Strahlung durch eine UV-durchlässige Wand aus Quarz in die Flüssigkeit abgibt. Die Vorrichtung umfasst ein Gehäuse, in dem sich die UV-Quellen und die Flüssigkeit befinden. Das Gehäuse kann rohrförmig sein und Körper/ Elemente aufweisen, die von der Mitte des Gehäuses radial nach außen ausgerichtet sind.

EP 2 567 713 A1 offenbart eine ähnliche Vorrichtung zum Entkeimen von Flüssigkeiten mittels mehreren UV-Quellen, die in Körper einer UV-durchlässigen Wand integriert sind. Die Wand ist von einem Gehäuse umfasst. Die UV-Quellen können in rohrförmigen Bereichen von Körpern innerhalb des Gehäuses eingeordnet sein.

Eine aus EP 0 818 206 A2 bekannte Vorrichtung zum Desinfizieren und Reinigen von Kleinteilen umfasst ein Gehäuse, das einen Behandlungsraum zur Aufnahme einer wässerigen oder alkoholischen Reinigungs-Lösung umschließt. Innerhalb des Gehäuses sind ein erster, einen ersten Entladungsraum aufweisender Excimer-Strahler zur Erzeugung von UV-Strahlung einer kürzeren Wellenlänge und ein zweiter, einen zweiten Entladungsraum aufweisender Excimer-Strahler zur Erzeugung von UV-Strahlung einer längeren Wellenlänge derart angeordnet, dass die von den Entladungsräumen ausgehende UV-Strahlung auf den Behandlungsraum einwirkt.

Der Erfindung liegt die Aufgabe zugrunde, eine Vorrichtung anzugeben, mit der das Bestrahlen beliebiger Gegenstände mit elektromagnetischer Strahlung mit hoher Wirksamkeit möglich ist.

Gelöst wird diese Aufgabe erfindungsgemäß mit einer Vorrichtung, welche die Merkmale von Anspruch 1 aufweist.

Bevorzugte und vorteilhafte Ausführungsformen der erfindungsgemäßen Vorrichtung sind Gegenstand der Unteransprüche.

Bei der erfindungsgemäßen Vorrichtung werden die zu bestrahlenden Gegenstände mit der elektromagnetischen Strahlung beaufschlagt, nachdem die Strahlung eine Wand aus für die anzuwendende Strahlung durchlässigem Werkstoff (z.B. Quarz für UV-Strahlung, oder Silizium, insbesondere kristallines Silizium, für IR-Strahlung) durchsetzt hat. Es ist also bei der erfindungsgemäßen Vorrichtung zwischen dem Gegenstand und der wenigstens einen Quelle für die Strahlung eine Wand, die als die Strahlung verteilender Lichtleiter wirkt, vorgesehen. Diese Wand kann als Rohr ausgebildet sein, in dem der Gegenstand oder die Gegenstände aufgenommen sind und das insbesondere mit einer Flüssigkeit, wie Wasser, gefüllt ist.

Bei der erfindungsgemäßen Vorrichtung sind in dem Raum, in dem der zu bestrahlende Gegenstand oder die zu bestrahlenden Gegenstände aufgenommen sind, Körper aus für elektromagnetische Strahlung in dem anzuwendenden Bereich durchlässigem Werkstoff (z.B. Quarzglas für UV-Strahlung, oder Silizium, insbesondere kristallines Silizium, für IR-Strahlung) vorgesehen. Den Körpern sind Strahlungsquellen zugeordnet. So ergibt sich eine gleichmäßige Verteilung der Strahlung in dem Raum, in dem die Gegenstände aufgenommen sind, sodass diese von allen Seiten gleichmäßig von der Strahlung getroffen werden und beispielsweise die gewünschte "Antifouling"-Eigenschaft der Gegenstände, insbesondere von mit Titandioxid beschichteten Fasern auf Keramik-Basis, entsteht.

Die erfindungsgemäße Vorrichtung erlaubt es aber auch, die Verteilung der Strahlung bewusst ungleichmäßig zu machen, damit Bereiche des Gegenstandes, die mehr Strahlung benötigen, verstärkt bestrahlt werden.

Die Vorrichtung besitzt ein Gehäuse, das rohrförmig ausgebildet ist.

Die Körper sind Leisten, die bevorzugt in Längsrichtung des (rohrförmigen) Gehäuses ausgerichtet sind. Die Strahlungsquellen sind beispielsweise den Schmalseiten der leistenförmigen Körper zugeordnet.

Bei der Erfindung sind Leisten vorgesehen, die von der Wand des Gehäuses nach innen abstehen.

In einer Ausführungsform sind weitere Leisten vorgesehen, die von der Mitte des Gehäuses nach außen weisend ausgerichtet sind. Die Strahlungsquellen, welche die elektromagnetische Strahlung (sichtbares Licht, IR-Strahlung oder UV-Strahlung) abgeben, sind den Körpern aus durchsichtigem Werkstoff zugeordnet.

Eine Ausführungsform der Erfindung zeichnet sich dadurch aus, dass die Außenfläche der mit der elektromagnetischen Strahlung bestrahlten Wand, insbesondere der Körper, der Vorrichtung aufgeraut ist. Ganz besonders bevorzugt ist eine Aufrauung mit Spitzen, sodass die Außenfläche der Wand und der Körper spitzrau ist.

Ein bevorzugter Werkstoff der Wand und der Körper der erfindungsgemäßen Vorrichtung, denen die wenigstens eine Strahlungsquelle zugeordnet ist, ist Quarzglas, das insbesondere geeignet ist, wenn als elektromagnetische Strahlung UV-Strahlung, also elektromagnetische Strahlung in einem nicht sichtbaren Bereich, abgegeben wird. Wenn in der erfindungsgemäßen Vorrichtung IR-Strahlung angewendet wird, ist ein bevorzugter Werkstoff der Wand oder der Körper der erfindungsgemäßen Vorrichtung, denen wenigstens eine Strahlungsquelle zugeordnet ist, Silizium, vorzugsweise kristallines Silizium.

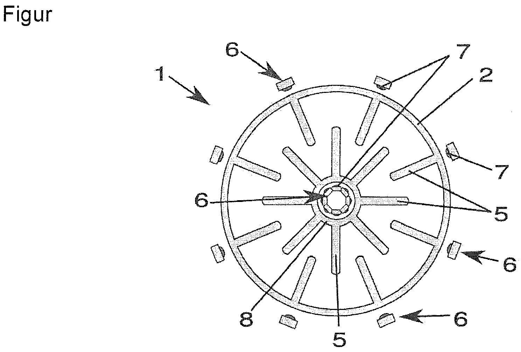

Weitere Einzelheiten und Merkmale der Erfindung ergeben sich aus der nachstehenden Beschreibung eines bevorzugten Ausführungsbeispiels anhand der Zeichnung, in der eine Ausführungsform einer erfindungsgemäßen Vorrichtung in Stirnansicht gezeigt ist.

Mit der erfindungsgemäßen Vorrichtung ist das Verteilen von elektromagnetischer Strahlung im sichtbaren und im nicht sichtbaren Bereich in Flüssigkeiten mit hoher Wirksamkeit möglich. So erlaubt es die erfindungsgemäße Vorrichtung, Rohre und Fasern, darunter auch Hohlfasern, mit elektromagnetischer Strahlung im sichtbaren und im nicht sichtbaren Bereich (UV-Strahlung oder IR-Strahlung) gleichmäßig und mit hoher Wirksamkeit zu bestrahlen.

Dank der besonderen Art der Einkoppelung von elektromagnetischer Strahlung im sichtbaren und im nicht sichtbaren Bereich in die als Lichtleiter dienende Wand, die beispielsweise in Form wenigstens eines als Lichtleiter dienenden Körpers ausgebildet ist und die aus für die gewählte Strahlung durchlässigem Werkstoff (Quarzglas oder Silizium), die, wenn die Wand rohrförmig ist, mit einer Flüssigkeit, z.B. Wasser, gefüllt ist oder im Falle der Körper in einer solchen Flüssigkeit angeordnet sind, ergibt sich die angestrebte Wirkung der erfindungsgemäßen Vorrichtung.

Mit der erfindungsgemäßen Vorrichtung ist es möglich, elektromagnetische Strahlung im sichtbaren, infraroten oder ultravioletten Bereich in einen mit Flüssigkeit gefüllten Behälter einzubringen, um Gegenstände zu bestrahlen.

Bevorzugte Lichtquellen sind LEDs, es können aber auch andere Lichtquellen, die elektromagnetische Strahlung im gewünschten Wellenlängenbereich abgeben, verwendet werden.

Insbesondere ist die erfindungsgemäße Vorrichtung geeignet, elektromagnetische Strahlung im sichtbaren, infraroten oder ultravioletten Bereich an ein Bündel faserförmiger Gegenstände, Röhrchen, Haare oder andere längliche Gegenstände abzugeben und gleichmäßig oder - falls in einem Anwendungsfall vorteilhaft - ungleichmäßig verteilt aufzubringen.

Die beiden äußeren Scheiben 11 tragen Stutzen 3 als Einlass bzw. Auslass. An die Stutzen 3 können Leitungen für das Zuführen und Abführen von Flüssigkeit (Wasser) angeschlossen werden.

Die in der Zeichnung in Stirnansicht gezeigte Ausführungsform umfasst das rohrförmige Gehäuse 2, von dem nach innen die Wand bildende Leisten 5 abstehen. Zusätzlich sind von einem die Wand bildenden rohrförmigen Bereich 8, der koaxial zum Gehäuse 2 angeordnet ist, nach außen abstehend Leisten 5 vorgesehen. Sowohl die nach innen als auch die nach außen abstehenden Leisten 5 sind 6 bezüglich der Achse des Gehäuses 2 radial ausgerichtet.

Bei der gezeigten Ausführungsform besteht im Rahmen der Erfindung die Möglichkeit, nur das Gehäuse 2 mit Leisten 5 oder, wie in der Zeichnung dargestellt, sowohl das Gehäuse 2 als auch den rohrförmigen Bereich 8 vorzusehen.

Die Leisten 5 bestehen beispielsweise aus Quarzglas und können mit dem Gehäuse 2 einstückig ausgebildet sein. Den Leisten 5, die vom Gehäuse 2 nach innen abstehen, sind außerhalb des Gehäuses 2 angeordnete LEDs 7 als elektromagnetische Strahlung abgebende Quellen 6 zugeordnet. Im Inneren des rohrförmigen Bereiches 8 sind mehrere LEDs 7 angeordnet, von denen jeweils eine den vom rohrförmigen Bereich 8 nach außen abstehenden Leisten 5, zugeordnet ist.

Die Leisten 5 haben eine Breite, die so groß ist, dass die freien Endbereiche der von außen nach innen und der von innen nach außen abstehenden Leisten 5 einander überlappen.

Die vom Gehäuse 2 nach innen abstehenden Leisten 5 können mit dem Gehäuse 2 einstückig ausgebildet sein. In diesem Fall besteht auch das Gehäuse 2 aus für die von den LEDs 7 abgegebene elektromagnetische Strahlung durchlässigem Werkstoff.

Bei der in der Zeichnung gezeigten Ausführungsformen einer erfindungsgemäßen Vorrichtung 1 sind die mit Strahlung zu behandelnden Gegenstände innerhalb des Gehäuses 2 zwischen den Leisten 5 angeordnet.

## Patentansprüche

1. Vorrichtung (1) zum Bestrahlen von Gegenständen, insbesondere von mit Titandioxid beschichteten Fasern, umfassend ein Gehäuse (2) und wenigstens eine Quelle (6) für die Abgabe elektromagnetischer Strahlung, wobei zwischen den in einer Flüssigkeit, wie Wasser, aufgenommenen Gegenständen und der Quelle (6) für die Abgabe elektromagnetischer Strahlung Wände aus für die elektromagnetische Strahlung durchlässigem Werkstoff, die als Lichtleiter wirken, vorgesehen sind, wobei den Wänden Quellen (6) für die elektromagnetische Strahlung zugeordnet sind, wobei das Gehäuse (2) rohrförmig ist und wobei die Wände als Leisten (5) aus für die von den Quellen (6) abgegebene elektromagnetische Strahlung durchlässigem Werkstoff ausgebildet sind, wobei die Leisten (5) bezüglich des Gehäuses (2) radial ausgerichtet sind, wobei die Leisten (5) von der Innenseite des Gehäuses (2) zu dessen Mitte hin weisend ausgerichtet sind.

2. Vorrichtung nach Anspruch 1, **dadurch gekennzeichnet, dass** das Gehäuse (2) oder die Leisten (5) aus Quarzglas oder aus Silizium besteht und die Quellen (6) UV-Strahlung oder IR-Strahlung abgeben.

3. Vorrichtung nach Anspruch 1 oder 2, **dadurch gekennzeichnet, dass** weitere Leisten (5) vorgesehen sind, die von der Mitte des Gehäuses (2) nach außen weisend ausgerichtet sind.

4. Vorrichtung nach Anspruch 3, **dadurch gekennzeichnet, dass** die nach innen und die nach außen weisenden Leisten (5) einander überlappen.

5. Vorrichtung nach einem der Ansprüche 1 bis 4, **dadurch gekennzeichnet, dass** die Quellen (6) für elektromagnetische Strahlung Rändern der Leisten (5) zugeordnet sind.

6. Vorrichtung nach einem der Ansprüche 1 bis 5, **dadurch gekennzeichnet, dass** Quellen (6) für elektromagnetische Strahlung außerhalb des Gehäuses (2) angeordnet sind.

## Claims

1. Device (1) for irradiating objects, in particular fibers coated with titanium dioxide, comprising a housing (2) and at least one source (6) for emitting electromagnetic radiation, wherein walls which consist of material that is permeable to the electromagnetic radiation and acts as a fiber optic waveguide are provided between the objects that are accommodated in a liquid, such as water, and the source (6) for emission of electromagnetic radiation, wherein sources (6) for the electromagnetic radiation are assigned to the walls, wherein the housing (2) is tubular, and wherein the walls are formed as strips (5) made from a material which is permeable to the electromagnetic radiation that is emitted by the sources (6), wherein the strips (5) are radially oriented with respect to the housing (2), wherein the strips (5) face the inside of the housing (2) towards the center thereof.

2. Device according to claim 1, **characterized in that** the housing (2) or the strips (5) consist of quartz glass or silicon and the sources (6) emit UV radiation or IR radiation.

3. Device according to claim 1 or 2, **characterized in that** further strips (5) are provided which are oriented outwards from the center of the housing (2).

4. Device according to claim 3, **characterized in that** the inwardly and outwardly facing strips (5) overlap each other.

5. Device according to one of the claims 1 to 4, **characterized in that** the sources (6) for electromagnetic radiation are assigned to edges of the strips (5).

6. Device according to one of claims 1 to 5, **characterized in that** sources (6) for electromagnetic radiation are arranged outside the housing (2).

## Revendications

1. Dispositif (1) pour irradier des objets, en particulier de fibres enrobées de dioxyde de titane, comprenant un boîtier (2) et au moins une source (6) pour l'émission de rayonnement électromagnétique, dans lequel sont prévues entre les objets placés dans un liquide tel que de l'eau et la source (6) pour l'émission de rayonnement électromagnétique des parois faites d'un matériau laissant passer le rayonnement électromagnétique qui agissent comme des guides d'ondes lumineuses, dans lequel les parois sont associées à des sources (6) du rayonnement électromagnétique, dans lequel le boîtier (2) est tubulaire et dans lequel les parois sont conformées comme des barres (5) de matériau laissant passer le rayonnement électromagnétique émis par les sources (6), les barres (5) étant orientées dans le sens radial par rapport au boîtier (2) et les barres (5) étant orientées de l'intérieur du boîtier (2) vers son milieu.

2. Dispositif selon la revendication 1, **caractérisé en ce que** le boîtier (2) ou les barres (5) se composent de sable de quartz ou de silicium et les sources (6) émettent un rayonnement UV ou un rayonnement IR.

3. Dispositif selon la revendication 1 ou 2, **caractérisé en ce que** sont prévues d'autres barres (5) qui sont orientées vers l'extérieur à partir du milieu du boîtier (2).

4. Dispositif selon la revendication 3, **caractérisé en ce que** les barres (5) orientées vers l'intérieur et l'extérieur se chevauchent les unes les autres.

5. Dispositif selon l'une des revendications 1 à 4, **caractérisé en ce que** les sources (6) de rayonnement électromagnétique sont associées à des bords des barres (5).

6. Dispositif selon l'une des revendications 1 à 5, **caractérisé en ce que** les sources (6) de rayonnement électromagnétique sont disposées à l'extérieur du boîtier (2).
